# EUROPEAN PATENT SPECIFICATION

(11) **EP 2 522 355 B1**
(45) Date of publication and mention of the grant of the patent: **21.02.2018**
(21) Application number: 10842249.4
(22) Date of filing: 28.12.2010
(51) Int. Cl.: A61K 35/745, A61P 17/00, A61P 43/00, A61Q 19/00, A61Q 19/08, A61K 8/99

(54) **DNA DAMAGE REPAIR PROMOTER FOR ORAL APPLICATION, AND ELASTASE ACTIVITY INHIBITOR FOR ORAL APPLICATION**
PROMOTER FÜR DIE REPARATUR VON DNA-SCHÄDEN ZUR ORALEN ANWENDUNG UND ELASTASEAKTIVITÄTSHEMMER ZUR ORALEN ANWENDUNG
PROMOTEUR DE RÉPARATION D'UN DOMMAGE À L'ADN POUR ADMINISTRATION ORALE, ET INHIBITEUR D'ACTIVITÉ D'ÉLASTASE POUR ADMINISTRATION ORALE

(30) Priority: 06.01.2010 JP 2010001576; 06.01.2010 JP 2010001550
(43) Date of publication of application: 14.11.2012
(73) Proprietor: Kabushiki Kaisha Yakult Honsha, Minato-ku Tokyo 105-8660 (JP)
(72) Inventor: SUGIMOTO, Saho, Tokyo 105-8660 (JP); SONE, Toshiro, Tokyo 105-8660 (JP); CHIBA, Katsuyoshi, Tokyo 105-8660 (JP)
(74) Representative: Blodig, Wolfgang
(86) International application number: PCT/JP2010/073796
(87) International publication number: WO 2011/083738

(56) References cited:
- EP-A1- 1 120 108
- WO-A1-03/070260
- JP-A- 57 500 830
- JP-A- 2002 326 946
- JP-A- 2004 510 740
- JP-A- 2005 525 348
- JP-A- 2006 076 926
- JP-A- 2010 006 757
- JP-A- 2010 006 757
- US-A1- 2002 168 388
- MITSUHARU MATSUMOTO: 'Health effects of metabolites produced by the consumption of probiotic strain LKM512' MILK SCIENCE vol. 58, no. 3, 2009, pages 143 - 152, XP008161240
- TSUKAHARA KAZUE ET AL.: 'The Effect of Sunscreen on Skin Elastase Activity Induced by Ultraviolet-A Irradiation' BIOLOGICAL & PHARMACEUTICAL BULLETIN vol. 28, no. 12, 2005, pages 2302 - 2307, XP008161222
- ALEX PINES ET AL.: 'Differential activity of UV-DDB in mouse keratinocytes and fibroblasts: Impact on DNA repair and UV-induced skin cancer' DNA REPAIR vol. 8, 2009, pages 153 - 161, XP025868971
- NAOKI MATSUDA ET AL.: 'UV-B Yuhatsu DNA Sonsho no Shufuku Oyobi Saiboshi ni Oyobosu IL-12 no Eikyo' PROCEEDINGS OF THE 47TH ANNUAL MEETING OF THE JAPAN RADIATION RESEARCH SOCIETY 2004, page 99, XP008161252

## Description

### Technical Field

The present invention relates to a DNA damage repair promoter for oral application and to an elastase activity suppressor for oral application.

### Background Art

DNA is damaged by various exogenous and endogenous causes, and such damage occurs always and continuously. In the long term, DNA damage impairs important functions such as replication and transcription and causes mutation, to thereby possibly cause cancer and aging.

Therefore, a living body has various repair mechanisms adapted to the types of DNA damage, with which DNA damage is continuously repaired, to thereby maintain genomic information and DNA functions. Examples of typical repair mechanisms include a homologous recombination repair mechanism against DNA double-strand break, a base excision repair mechanism against oxidative base damage by active oxygen species, a nucleotide excision repair mechanism against pyrimidine dimer formed by UV light, and a mismatch repair mechanism against replication errors.

However, abnormality or impairment of such a repair mechanism or strong damage beyond the repair capacity may occur in some cases for a certain reason. In such cases, cells are prone to die via apoptosis, and mutation is induced, thereby promoting a long-term process of cancer, aging, or the like.

Under such circumstances, materials for mitigating such damage factors have been developed. Specifically, there have been reported cases of suppression of DNA damage in the large intestine and liver caused by a carcinogen. More specifically, *Lactobacillus gasseri* (P79), *Lactobacillus confusus* (DSM20196), *Streptococcus thermophilus* (NCIM50083), *Bifidobacterium breve,* or *Bifidobacterium longum* suppresses DNA damage in the large intestine caused by N-methyl-N'-nitro-N-nitrosoguanidine (Non-Patent Document 1); a milk fermented by *Bifidobacterium animalis* DN-173 010 or *Streptococcus thermophilus* DN-001 158 suppresses DNA damage in the large intestine caused by a heterocyclic aromatic amine (Non-Patent Document 2); and *Lactobacillus vulgaris 291, Streptococcus thermophilus* F4, *Streptococcus thermophilus* V3, or *Bifidobacterium longum* BB536 suppresses DNA damage in the large intestine and liver caused by a heterocyclic aromatic amine (Non-Patent Document 3).

However, suppression of DNA damage differs from repair of DNA damage. The aforementioned suppression of DNA damage caused by a carcinogen is attained by causing perorally taken bacteria to be adsorbed on the surface of the digestive tract, or by causing a carcinogen to be adsorbed on bacterium cells and excreting the carcinogen therewith, to thereby suppress contact of perorally taken carcinogens to the body and absorption of them by the body. Thus, such suppression is considered to fail to promote repair of DNA damage.

Meanwhile, UV-ray-induced DNA damage is caused by cyclobutane pyrimidine dimer or a 6-4-type photoreaction product formed in skin cells through exposure to UV light. To cope with increased amount of UV radiation attributed to depletion of the ozone layer in years, means for prevention and suppression of the DNA damage have been developed.

One known DNA damage prevention method is absorption or scattering of UV light by use of a sunscreen. Through employment of this method, only a UV-ray-induced skin disorder can be mitigated, but repair of DNA damage caused by exposure to UV light cannot be promoted. Also, one known cosmetic composition for suppressing DNA damage caused by exposure to UV light comprises a first ingredient which is an inactivated culture of a bacterium belonging to the genus *bifidobacterium,* and a second ingredient which is a plant extracellular matrix extract composed of glucoprotein, carbohydrate polymer, and arabinogalactan protein, and is used for topical administration against UV-radiation-induced skin damage (Patent Document 1).

Meanwhile, exposure to UV light results in DNA damage as well as photo-aging of the skin such as wrinkle formation or reduction in elasticity. Although the mechanism of photo-aging has not been elucidated completely, variation in dermal extracellular matrix composition is thought to be an important factor. Dermal extracellular matrix ingredients include collagen, elastin, and glycosaminoglycan. Among them, elastin is a main protein forming elastic fiber.

It has been reported that the activities of neutrophil elastase and fibroblast elastase, which are elastin protease, are enhanced in the skin exposed to UV light. The changes in amount and property of elastin caused by these elastases are thought to cause wrinkle formation and reduction in elasticity.

For the purpose of skin anti-aging or revitalization, a variety of elastase activity inhibitors have been proposed. For example, there have been known an elastase activity inhibitor containing a fermented product of perilla leaves (Patent Document 2), that containing a fermented product of parsley (Patent Document 3), and that containing a fermented product of green pepper (Patent Document 4), which are used for skin care purpose.

The elastase activity inhibition was confirmed by an in vitro test in which elastase activity was measured in a solution of elastase derived from human neutrophils or pig pancreas to which solution a test substance had been added. Thus, the elastase activity inhibition has never been confirmed by an in vivo test. In order for the substance actually applied to the skin to have elastase activity suppressing action, an active ingredient must penetrate the horny layer and the epidermis and reach the dermis. Thus, there is demand for an elastase activity suppressor whose suppressing action has been confirmed in vivo.

### Prior Art Documents

### Patent Documents

Patent Document 1: JP-A-2002-255777
Patent Document 2: JP-A-2006-61091
Patent Document 3: JP-A-2006-75085
Patent Document 4: JP-A-2006-76926

### Non-Patent Documents

Non-Patent Document 1: Pool-Zobel BL. et al., Nutr. Cancer, 26, 365-80, 1996
Non-Patent Document 2: Tavan E. et al., Carcinogenesis., 23, 477-83, 2002
Non-Patent Document 3: Zsivkovits M. et al., Carcinogenesis., 24, 1913-1918, 2003

### Summary of the Invention

### Problems to be Solved by the Invention

However, there have never been known that promotion of DNA damage repair and elastase activity suppression can be attained through perorally administering a bacterium belonging to the genus *bifidobacterium.*

An object of the present invention is to provide a DNA damage repair promoter for oral application and an elastase activity suppressor for oral application.

### Means for Solving the Problems

The present inventors have conducted extensive studies on promotion of DNA damage repair and suppression of elastase activity. As a result, quite surprisingly, the inventors have found that DNA damage repair can be promoted and elastase activity can be suppressed through peroral ingestion of a bacterium belonging to the genus *bifidobacterium.*
i) Accordingly, the present invention provides a DNA damage repair promoter for oral application containing, as an active ingredient, a bacterium belonging to the genus *bifidobacterium.*
ii) The present invention also provides a cyclobutane pyrimidine dimer level reduction promoter for oral application containing, as an active ingredient, a bacterium belonging to the genus *bifidobacterium.*
iii) The present invention also provides an elastase activity suppressor for oral application containing, as an active ingredient, a bacterium belonging to the genus *bifidobacterium.*
iv) The present invention also provides an elastase activity suppressor as described in iii) above, which is a skin anti-aging and revitalizing agent.
v) The present disclosure also provides a method for promoting DNA damage repair, comprising perorally administering, to a subject in need thereof, a bacterium belonging to the genus *bifidobacterium.*
vi) The present disclosure also provides a method for promoting reduction of cyclobutane pyrimidine dimer level, comprising perorally administering, to a subject in need thereof, a bacterium belonging to the genus *bifidobacterium.*
vii) The present invention also provides a non-therapeutic method for suppressing elastase activity, comprising perorally administering, to a subject in need thereof, a bacterium belonging to the genus *bifidobacterium.*
viii) The present invention also provides a method as described in vii) above, which is a skin anti-aging and revitalizing method.
ix) The present invention also provides a bacterium belonging to the genus *bifidobacterium* for use in promoting DNA damage repair through oral administration thereof.
x) The present invention also provides a bacterium belonging to the genus *bifidobacterium* for use in promoting reduction of cyclobutane pyrimidine dimer level through oral administration thereof.
xi) The present invention also provides a bacterium belonging to the genus *bifidobacterium* for use in suppressing elastase activity through oral administration thereof.
xii) The present invention also provides a bacterium as described in xi) above, for use in anti-aging and revitalizing the skin.
xiii) The present invention also provides use of a bacterium belonging to the genus *bifidobacterium* for the manufacture of a DNA damage repair promoter for oral application.
xiv) The present invention also provides use of a bacterium belonging to the genus *bifidobacterium* for the manufacture of a cyclobutane pyrimidine dimer level reduction promoter for oral application.
xv) The present invention also provides use of a bacterium belonging to the genus *bifidobacterium* for the manufacture of an elastase activity suppressor for oral application.
xvi) The present invention also provides use as described in xv) above for the manufacture of an anti-aging and revitalizing agent.

The invention is defined in the claims.

### Effects of the Invention

According to the present invention, DNA damage repair can be promoted through peroral administration of a relevant agent. Therefore, the DNA damage repair promoter for oral application of the present invention is useful for producing pharmaceuticals, foods and beverages, etc. for promoting DNA damage repair.

Also, according to the present invention, elastase activity can be suppressed through peroral administration of a relevant agent. Therefore, the elastase activity suppressor for oral application of the present invention is useful for producing pharmaceuticals, foods and beverages, etc. for anti-aging and revitalizing the skin.

### Brief Description of the Drawings

[Fig. 1] A graph showing that repair of DNA damage can be promoted through peroral administration of cells of a bacterium.
[Fig. 2] A graph showing that repair of DNA damage can be promoted through peroral administration of cells of a bacterium.
[Fig. 3] A graph showing that elastase activity can be suppressed through peroral administration of cells of a bacterium.
[Fig. 4] A graph showing that elastase activity can be suppressed through peroral administration of cells of a bacterium.

### Modes for Carrying Out the Invention

The active ingredient of the DNA damage repair promoter for oral application of the present invention (hereinafter may be referred to simply as a "DNA damage repair promoter") or the elastase activity suppressor for oral application of the present invention (hereinafter may be referred to simply as an "elastase activity suppressor") is a bacterium belonging to the genus *bifidobacterium.* Firstly, the bacterium belonging to the genus *bifidobacterium* will be described in detail.

Examples of the aforementioned bacterium belonging to the genus *bifidobacterium* include *Bifidobacterium breve, Bifidobacterium bifidum, Bifidobacterium longum, Bifidobacterium adolescentis, Bifidobacterium catenulatum, Bifidobacterium pseudocatenulatum,* and *Bifidobacterium animalis.* These bacteria may be used singly or in combination of two or more species. Among them, *Bifidobacterium breve* is preferred, from the viewpoints of DNA damage repair promoting action and elastase activity suppressing action.

The *Bifidobacterium breve* is preferably *Bifidobacterium* breve YIT 4063 (FERM BP-2823), *Bifidobacterium breve* YIT 4064 (FERM BP-2824), *Bifidobacterium breve* YIT 4065 (FERM BP-6223) (deposited to the International Patent Organism Depositary, National Institute of Advanced Industrial Science and Technology (former: National Institute of Bioscience and Human-Technology, Agency of Industrial Science and Technology, Ministry of International Trade and Industry), Address of depositary institution: 1-1-3 Higashi, Tsukuba, Ibaraki 305 Japan, Date of deposit: Feb. 29, 1996), *Bifidobacterium breve* YIT 12272 (FERM BP-11320) (deposited to the International Patent Organism Depositary, National Institute of Advanced Industrial Science and Technology, Address of depositary institution: Central 6, 1-1-1 Higashi, Tsukuba, Ibaraki 305-8566 Japan, Date of deposit: Feb. 16, 2010), and descendant strains thereof. Of these, *Bifidobacterium breve* YIT 4065 and *Bifidobacterium breve* YIT 12272 are particularly preferred, from the viewpoints of DNA damage repair promoting action and elastase activity suppressing action. As used herein, the term "descendant strain" refers to a concept encompassing naturally occurring mutants, mutants obtained through mutation treatment, and genetically modified mutants, and the like.

The aforementioned bacterium belonging to the genus *bifidobacterium* may be in the form of cells of bacterium (live bacterium) or a processed product of the bacterium cells. No particular limitation is imposed on the processed product, so long as the product is obtained through a conventional processing method. Examples of such a processed product include heated cells (killed bacterium cells), a lyophilized product thereof, a culture containing any of these, a cell broken liquid (e.g., ultrasonicated liquid), an enzyme-treated liquid of bacterium cells, and a solid residue obtained through solid-liquid phase separation (e.g., filtration or centrifugation) of such a cell product; a cell-wall-removed (by an enzyme or a machine) processed liquid, a concentrate of the processed liquid, a diluted product thereof, and a dried product thereof; a nucleic-acid-containing fraction obtained through dissolving bacterium cells with a surfactant or the like and precipitating with ethanol or the like; and a separated/purified (by, for example chromatography) product of the cell broken liquid (e.g., ultrasonicated liquid), the enzyme-treated liquid of bacterium cells, etc.

The aforementioned bacterium belonging to the genus *bifidobacterium* is preferably in the form of cells of bacterium (live bacterium), heated cells (killed bacterium cells), a lyophilized product thereof, a cell broken liquid (e.g., ultrasonicated liquid), or an enzyme-treated liquid of bacterium cells. Of these, cells of bacterium (live bacterium) and the lyophilized product thereof are particularly preferred, from the viewpoints of DNA damage repair promoting action and elastase activity suppressing action.

Notably, the killed bacterium cells may be produced through heating, a drug treatment (e.g., treatment with an antibiotic substance), a chemical treatment (e.g., treatment with formalin), a UV treatment, or a radiation (e.g., γ-ray) treatment.

Next, the "DNA damage repair promoter" of the present invention will be described in detail.

In the present invention, the term "promotion of DNA damage repair" refers to promotion of repair of damaged DNA molecules. As shown in the Examples hereinbelow, the bacterium belonging to the genus *bifidobacterium* of the present invention promotes reduction of the cyclobutane pyrimidine dimer (hereinafter abbreviated as CPD) level through peroral administration thereof. Therefore, the bacterium has excellent DNA damage repair promoting action.

Thus, the bacterium belonging to the genus *bifidobacterium* may be used as a DNA damage repair promoter and for the manufacture of a DNA damage repair promoter.

Since cancer and aging can be prevented and treated by promoting repair of DNA damage, the bacterium belonging to the genus *bifidobacterium* may also serve as a cancer or aging prophylactic and therapeutic agent. The aforementioned DNA damage repair promoter is useful as a pharmaceutical product, a quasi-drug, foods and beverages, pet foods, etc. for preventing or treating cancer or aging of humans and animals.

Since the DNA damage repair promoter of the present invention can promote repair of DNA damage caused by exposure to UV light, the bacterium belonging to the genus *bifidobacterium* may also serve as a skin cancer or skin aging prophylactic and therapeutic agent.

As described above, the bacterium belonging to the genus *bifidobacterium* of the present invention has a CPD level reduction promoting action. Thus, the bacterium belonging to the genus *bifidobacterium* may serve as a CPD level reduction promoter and may be used in production of a CPD level reduction promoter.

Since the CPD level reduction promoter of the present invention can reduce CPD level, which is an index for DNA damage, the bacterium belonging to the genus *bifidobacterium* may also serve as a cancer or aging prophylactic and therapeutic agent. Since CPD is formed by exposure to UV light, the CPD level reduction promoter of the present invention is particularly useful as a skin cancer or skin aging prophylactic and therapeutic agent.

Next, the "elastase activity suppressor" of the present invention will be described in detail.

As described in the Examples hereinbelow, the bacterium belonging to the genus *bifidobacterium* of the present invention can suppress, through peroral administration thereof, elastase activity in the skin which has been enhanced through exposure to UV light. Therefore, the bacterium belonging to the genus *bifidobacterium* may serve as an elastase activity suppressor and may be used in production of an elastase activity suppressor.

Suppression of elastase activity results in skin anti-aging and revitalization of the skin. Thus, the bacterium belonging to the genus *bifidobacterium* may also serve as a skin anti-aging and revitalizing agent. The elastase activity suppressor is useful as a pharmaceutical product, a quasi-drug, foods and beverages, pet foods, etc. for skin anti-aging or skin revitalizing of humans and animals. Notably, the term "skin anti-aging and revitalizing" encompasses prevention and amelioration of wrinkles of the skin, prevention of skin sag, improvement of skin elasticity, and anti-aging.

Next, modes of employment of the aforementioned DNA damage repair promoter, elastase activity suppressor, and skin anti-aging and revitalizing agent will be described.

The DNA damage repair promoter, elastase activity suppressor, or skin anti-aging and revitalizing agent is perorally administered. The administration may be performed before, during, or after exposure to UV light. In order to fully attain DNA damage repair promoting action, elastase activity suppressing action, and skin anti-aging and revitalizing action, each of the agents is preferably administered at least before exposure to UV light. More preferably, the agent is administered before and during exposure to UV light. In the case where the administration starts before exposure to UV light, the administration period before the exposure is preferably five days or longer, more preferably 5 to 10 days, in order to fully attain DNA damage repair promoting action, elastase activity suppressing action, and skin anti-aging and revitalizing action. When the administration is performed continuously for 10 days or longer, satisfactory DNA damage repair promoting action, elastase activity suppressing action, and skin anti-aging and revitalizing action are ensured.

In the case where the DNA damage repair promoter, elastase activity suppressor, or skin anti-aging and revitalizing agent is used as a pharmaceutical product, examples of the peroral dosage form include tablet, capsule, granules, coated tablet, pill, fine granules, powder, powdery agent, sustained-release agent, suspension, emulsion, syrup, freeze-dried agent, liquid, and elixir.

The above pharmaceuticals may be produced through a generally employed method. The bacterium belonging to the genus *bifidobacterium* may be used as a single ingredient or may be combined with a pharmaceutically acceptable carrier. Examples of the carrier include a vehicle, a binder, a disintegrant, a surfactant, a lubricant, a fluidity-enhancer, a flavoring agent, a colorant, a perfume, a diluent, a disinfectant, an osmolyte, a pH-regulator, an emulsifier, an antiseptic, a stabilizer, an absorption aid, an anti-oxidant, a UV-absorber, a humectant, a thickener, a glazing agent, an activity-enhancer, an anti-inflammatory agent, a tonicity agent, a soothing agent, and an odor-improver.

Examples of the binder include starch, dextrin, acacia powder, gelatin, methylcellulose, hydroxypropylcellulose, crystalline cellulose, ethylcellulose, polyvinylpyrrolidone, and Macrogol.

Examples of the disintegrant include hydroxypropyl starch, sodium carboxymethylcellulose, calcium carboxymethylcellulose, carboxymethylcellulose, and low-substituted hydroxypropylcellulose.

Examples of the surfactant include sodium lauryl sulfate, soybean lecithin, sucrose fatty acid ester, and Polysolvate 80.

Examples of the lubricant include talc, waxes, hydrogenated vegetable oil, magnesium stearate, calcium stearate, aluminum stearate, and polyethylene glycol.

Examples of the fluidity-enhancer include light anhydrous silicic acid, aluminum hydroxide dry gel, synthetic aluminum silicate, and magnesium silicate.

Examples of the diluent include distilled water for injection, physiological saline, aqueous glucose, olive oil, sesame oil, peanut oil, soybean oil, corn oil, propylene glycol, and polyethylene glycol.

The DNA damage repair promoter, elastase activity suppressor, and skin anti-aging and revitalizing agent of the present invention are used not only as pharmaceutical products as described above, but also as foods and beverages, quasi-drugs, pet foods, etc. In the latter case, the bacterium belonging to the genus *bifidobacterium* alone or a mixture thereof with various nutrient compositions is incorporated into any of the foods and beverages and the like. The thus-prepared foods and beverages may be used as health foods or food materials useful for preventing or treating cancer or aging, or for skin-anti-aging and revitalizing. To these foods and beverages or containers thereof, a label indicating the aforementioned effect(s) may be attached.

In the case where the DNA damage repair promoter, elastase activity suppressor, or skin anti-aging and revitalizing agent is incorporated into foods and beverages, additives which are acceptable to foods and beverages are appropriately used, and the mixtures are processed into appropriately edible forms through conventional means. Examples of the form include granules, grains, tablet, capsule, and paste. The agent may be used in various foods such as processed meat products (e.g., ham and sausage), processed fish products (e.g., *kamaboko* and *chikuwa*), bread, confectionary, butter, powdered milk, and fermented foods and beverages. Alternatively, the agent may be added to beverages such as water, fruit juice, milk, refreshing beverages, and tea beverages. Among these foods and beverages, preferred are fermented foods and beverages each containing the bacterium belonging to the genus *bifidobacterium* serving as an active ingredient (e.g., fermented milk, lactic acid bacteria beverage, fermented soy milk, fermented fruit juice, and fermented plant liquid).

These fermented foods and beverages may be produced through a generally employed method. For example, in one production procedure of a fermented milk, a bacterium belonging to the genus *bifidobacterium* is cultured in a sterilized milk medium, and the medium is homogenized, to thereby produce a fermented milk base. Subsequently, a syrup prepared separately is added to the milk base under mixing. The mixture is further homogenized by means of a homogenizer or the like, and a flavor is added thereto, to thereby produce a final product. The thus-produced fermented milk may have any form such as a plane type, a soft type, a fruit flavor type, solid or liquid.

Next, the dose of the bacterium belonging to the genus *bifidobacterium* and other conditions will be described.

No strict limitation is imposed on the dose of the bacterium belonging to the genus *bifidobacterium,* which is an active ingredient of the DNA damage repair promoter, elastase activity suppressor, or skin anti-aging and revitalizing agent of the present invention. Preferably, an appropriate dose is determined, since the effect of the agent varies in accordance with the mode of employment (e.g., target subject or target disease). In order to fully attain the DNA damage repair promoting action, elastase activity suppressing action, and skin anti-aging and revitalizing action, the daily dose (cell count) of the bacterium belonging to the genus *bifidobacterium* is preferably 1 × 10³ CFU or higher, more preferably 1 × 10³ to 1 × 10¹³ CFU, particularly preferably 1 × 10⁶ to 1 × 10¹⁰ CFU.

Meanwhile, no particular limitation is imposed on the wavelength of the aforementioned UV ray. A UV ray having a wavelength of 280 to 400 nm has a high probability of causing DNA damage, finally resulting in skin cancer and skin aging. In addition, since such a UV ray enhances elastase activity, to thereby possibly cause skin aging, the DNA damage repair promoter, elastase activity suppressor, and skin anti-aging and revitalizing agent of the present invention are suitably employed as a DNA damage repair promoter, an elastase activity enhancement suppressor, and a skin anti-aging and revitalizing agent, whose target phenomena are caused through exposure to a UV ray or UV rays having a wavelength of 280 to 400 nm. No particular limitation is imposed on the UV dose. When the daily dose is 20 mJ/cm² or higher, particularly 40 mJ/cm² or higher, the DNA damage repair promoter, elastase activity suppressor, and skin anti-aging and revitalizing agent of the present invention are suitably employed.

Also, the DNA damage repair promoter and cyclobutane pyrimidine dimer level reduction promoter of the present invention are designed to be perorally administered for intake of the bacterium belonging to the genus *bifidobacterium,* which have long been used in foods. Thus, as compared with cancer chemotherapy, in which an anti-cancer agent is administered through typically employed intravenous injection or the like associated with severe adverse effects, the promoters of the invention have remarkably higher safety and can considerably reduce the distress of a subject in need thereof.

### Examples

The present invention will next be described in detail by way of examples, which should not be construed as limiting the invention thereto. Unless otherwise specified, in the following Examples, the unit "%" means a unit "mass/vol.%."

### Example 1 (Preparation of bacterium cell solution)

The medium disclosed by Rogosa et al. (Eftymiou C. et al., J. Infect. Dis., 110, 258-267, 1962) was modified to have the following composition, and the modified medium was sterilized by heating at 121°C for 15 minutes. To the sterilized medium, cells of *Bifidobacterium breve* YIT 4065 (FERM BP-6223) were inoculated at 1 v/v%, and anaerobically cultured at 37°C for about 20 hours. The thus-obtained culture liquid was centrifuged at 3,500×G, to thereby recover cells of a bacterium belonging to the genus *bifidobacterium.* The cells were suspended in physiological saline, to thereby prepare a bacterium cell solution having a cell concentration of 1.0 × 10¹⁰ CFU/mL.

Composition of the medium:
trypticase: 1%, yeast extract: 0.5%, tryptose: 0.3%, potassium phosphate(I): 0.3%, potassium phosphate(II): 0.39%, ammonium citrate: 0.2%, lactose: 1%, L-cysteine hydrochloride: 0.03%, Tween 80: 0.1%, and a salt solution (MgSO₄·7H₂O: 11.5 g, FeSO₄·7H₂O: 0.68 g, and MnSO₄·2H₂O: 2.4 g dissolved in water (100 mL)): 0.5%.

### Test Example 1 (CPD level test)

The DNA repair promotion effect of the bacterium cell solution was evaluated by measuring the CPD level of a sample.

Hair-less mice (Hos: HR-1 (6-week-old)) were acclimated for one week and divided into eight groups (n=6): group 1 to group 8.

Physiological saline was perorally administered to groups 1, 3, 5, and 7 (hereinafter may be referred to as control groups). To groups 2, 4, 6, and 8 (hereinafter referred to as bacterium cell groups), a bacterium cell solution prepared in Example 1 was perorally administered. In all cases, the solution was administered to mice at a dose of 0.1 mL/day for nine days.

Groups 1 and 2 were not exposed to UV light. Groups 3 to 8 were irradiated with UV light (including 280 to 400 nm) at a daily dose of 40 mJ/cm² by means of a UV-radiation apparatus (Toshiba SE-FL-20) for four days from day 6 after start of administration, while the administration was continued.

From each mouse, the dorsal skin was removed 24 hours after the last administration (groups 1 and 2), 6 hours after the last irradiation (groups 3 and 4), 12 hours after the last irradiation (groups 5 and 6), or 24 hours after the last irradiation (groups 7 and 8).

In the following Table 1, "UV+" denotes performing UV irradiation, and "UV-" denotes performing no UV irradiation.

**[Table 1]**

| | |
|---|---|
| UV-free | Group 1: control group (UV-, physiological saline) |
| | Group 2: bacterium cell group (UV-, bacterium cells) |
| Skin collection 6 hr after the last UV exposure | Group 3: control group (UV+, physiological saline) |
| | Group 4: bacterium cell group (UV+, bacterium cells) |
| Skin collection 12 hr after the last UV exposure | Group 5: control group (UV+, physiological saline) |
| | Group 6: bacterium cell group (UV+, bacterium cells) |
| Skin collection 24 hr after the last UV exposure | Group 7: control group (UV+, physiological saline) |
| | Group 8: bacterium cell group (UV+, bacterium cells) |

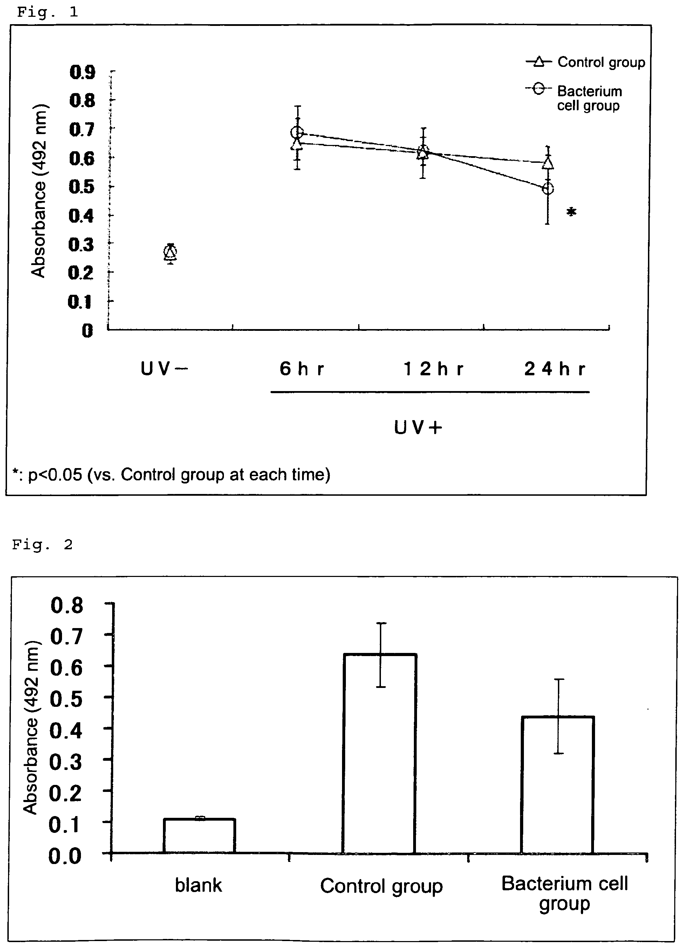

From each of the removed dorsal skin samples, genomic DNA was purified (QIAamp (registered trademark) DNA mini kit). An aliquot of the genomic DNA was applied to a 96-well plate, and a cyclobutane pyrimidine dimer antibody (TDM-2) was bound to the genomic DNA. Subsequently, the signal was amplified with a biotin-labeled secondary antibody and enzyme-labeled streptavidine. The plate was colored by adding a substrate thereto, and the absorbance was measured at 492 nm (ELISA). Fig. 1 shows the results.

As shown in Fig. 1, the skin sample CPD level of the control group (group 3), as measured six hours after the last UV irradiation, was almost the same as that of the bacterium cell group (group 4). However, the skin sample CPD level of the bacterium cell group (group 8), as measured 24 hours after the last UV irradiation, was significantly lower than that of the control group (group 7). Therefore, repair of DNA damage caused by UV light was found to be promoted through administration of *Bifidobacterium breve* YIT 4065 (FERM BP-6223).

### Example 2 (Preparation of bacterium cell sample)

A medium was prepared from mineral solution (1 w/v%), yeast extract (1 w/v%), lactose (3 w/v%), and milk protein (5 w/v%). The medium (1.5 L) was added to a 2L flask and sterilized by heating at 121°C for 15 minutes. To the sterilized medium, cells of *Bifidobacterium breve* YIT 12272 (FERM BP-11320) were inoculated at 1 v/v%, and anaerobically cultured at 36°C for about 20 hours, while the pH of the culture was maintained at 5.5 by use of sodium hydroxide. The thus-obtained culture liquid was centrifuged at 15,000×G, to thereby recover cells of a bacterium belonging to the genus *bifidobacterium.* The above mineral solution had the following composition: potassium phosphate(I) (10 w/v%), potassium phosphate(II) (20 w/v%), sodium acetate (30 w/v%), and ammonium sulfate (30 w/v%).

Separately, a dispersion (100 mL) of milk protein (8 w/v%) and sugar (4 w/v%) was prepared and sterilized by heating at 121°C for 15 minutes. To the dispersion, the above collected cells of the bacterium belonging to the genus *bifidobacterium* were dispersed at 15% (based on wet weight) and then lyophilized, to thereby yield freeze-dried cells of *Bifidobacterium breve* YIT 12272 (FERM BP-11320). The freeze-dried cells were suspended in physiological saline (10 mL), to thereby prepare a bacterium cell sample having a cell concentration of 4 × 10⁹ CFU/mL.

### Test Example 2 (CPD level test)

Hair-less mice (Hos: HR-1 (6-week-old)) were acclimated for one week and divided into three groups (n=3): group 1 to group 3.

Physiological saline was perorally administered to groups 1 and 2. To group 3, a bacterium cell sample prepared in Example 2 was perorally administered. In all cases, the solution was administered to mice at a dose of 0.1 mL/day for nine days.

Group 1 was not exposed to UV light. Groups 2 and 3 were irradiated with UV light (including 280 to 400 nm) at a daily dose of 50 mJ/cm² by means of a UV-radiation apparatus (Toshiba SE-FL-20) for four days from day 6 after start of administration, while the administration was continued. Then, from each mouse, the dorsal skin was removed.

Among the mouse groups, a group to which physiological saline was administered but which was not irradiated with UV light was a blank group; a group to which physiological saline was administered and which was irradiated with UV light was a control group; and a group to which a bacterium cell sample was administered and which was irradiated with UV light was a bacterium cell group (Table 2). In the following Table 2, "UV+" denotes performing UV irradiation, and "UV-" denotes performing no UV irradiation.

**[Table 2]**

| | |
|---|---|
| Group 1 | Blank group (UV-, physiological saline) |
| Group 2 | Control group (UV+, physiological saline) |
| Group 3 | Bacterium cell group (UV+, bacterium cells) |

From each of the removed dorsal skin samples, genomic DNA was purified (QIAamp (registered trademark) DNA mini kit). An aliquot of the genomic DNA was applied to a 96-well plate, and a cyclobutane pyrimidine dimer antibody (TDM-2) was bound to the genomic DNA. Subsequently, the signal was amplified with a biotin-labeled secondary antibody and enzyme-labeled streptavidine. The plate was colored by adding a substrate thereto, and the absorbance was measured at 492 nm (ELISA). Fig. 2 shows the results.

As shown in Fig. 2, the skin sample CPD level of the control group was significantly elevated through exposure to UV light, as compared with that of the blank group. In contrast, the skin sample CPD level of the bacterium cell group was lowered, as compared with that of the control group. Therefore, repair of DNA damage caused by UV light was found to be promoted through administration of *Bifidobacterium breve* YIT 12272 (FERM BP-11320).

### Example 3 (Preparation of bacterium cell sample)

A medium was prepared from mineral solution (1 w/v%), yeast extract (1 w/v%), lactose (3 w/v%), and milk protein (5 w/v%). The medium (1.5 L) was added to a 2L flask and sterilized by heating at 121°C for 15 minutes. To the sterilized medium, cells of *Bifidobacterium breve* YIT 4065 (FERM BP-6223) were inoculated at 1 v/v%, and anaerobically cultured at 36°C for about 20 hours, while the pH of the culture was maintained at 5.5 by use of sodium hydroxide. The thus-obtained culture liquid was centrifuged at 15,000×G, to thereby recover cells of a bacterium belonging to the genus *bifidobacterium.* The above mineral solution had the following composition: potassium phosphate(I) (10 w/v%), potassium phosphate(II) (20 w/v%), sodium acetate (30 w/v%), and ammonium sulfate (30 w/v%).

Separately, a dispersion (100 mL) of milk protein (8 w/v%) and sugar (4 w/v%) was prepared and sterilized by heating at 121°C for 15 minutes. To the dispersion, the above collected cells of the bacterium belonging to the genus *bifidobacterium* were dispersed at 15% (based on wet weight) and then lyophilized, to thereby yield freeze-dried cells of *Bifidobacterium breve* YIT 4065 (FERM BP-6223). The freeze-dried cells were suspended in physiological saline (10 mL), to thereby prepare a bacterium cell sample having a cell concentration of 1.0 × 10¹⁰ CFU/mL.

### Test Example 3 (Elastase activity suppression test)

Hair-less mice (Hos: HR-1 (6-week-old)) were acclimated for one week and divided into three groups (n=5): group 1 to group 3.

Physiological saline was perorally administered to groups 1 and 2. To group 3, a bacterium cell sample prepared in Example 3 was perorally administered. In all cases, the solution was administered to mice at a dose of 0.1 mL/day for nine days.

Group 1 was not exposed to UV light. Groups 2 and 3 were irradiated with UV light (including 280 to 400 nm) at a daily dose of 40 mJ/cm² by means of a UV-radiation apparatus (Toshiba SE-FL-20) for four days from day 6 after start of administration, while the administration was continued. Then, from each mouse, the dorsal skin was removed.

Among the mouse groups, a group to which physiological saline was administered but which was not irradiated with UV light was a blank group; a group to which physiological saline was administered and which was irradiated with UV light was a control group; and a group to which a bacterium cell sample was administered and which was irradiated with UV light was a bacterium cell group (Table 3). In the following Table 3, "UV+" denotes performing UV irradiation, and "UV-" denotes performing no UV irradiation.

**[Table 3]**

| | |
|---|---|
| Group 1 | Blank group (UV-, physiological saline) |
| Group 2 | Control group (UV+, physiological saline) |
| Group 3 | Bacterium cell group (UV+, bacterium cells) |

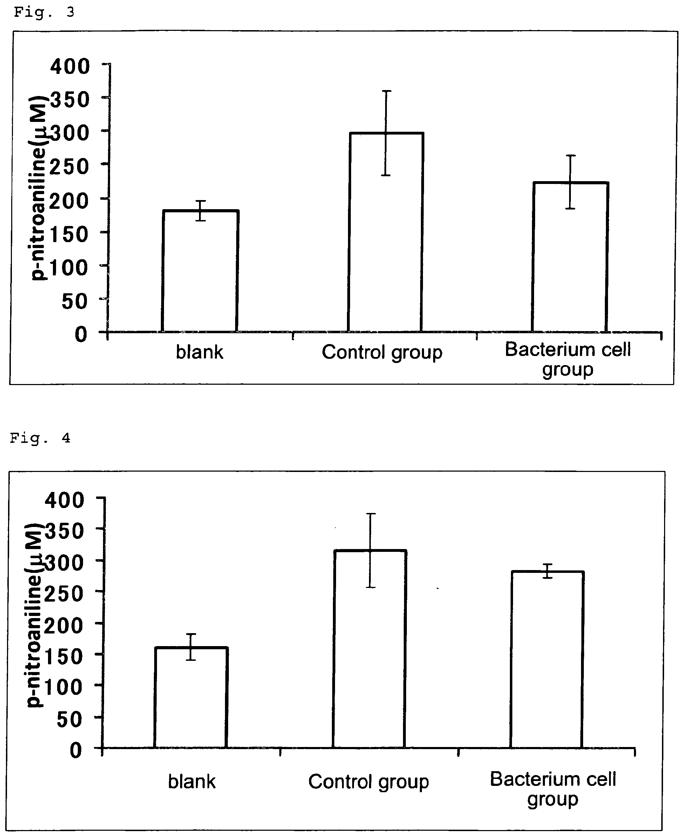

To each of the thus-collected dorsal skin samples, 50 mM Tris-HCl (pH: 7.5) was added, and the mixture was homogenized by means of Polytron. The homogenate was centrifuged at 14,000×G, and the supernatant was dispensed to a 96-well plate (each 100 µL). Instead of elastin, 6.25 mM succinyl-L-alanyl-L-alanyl-L-alanine-p-nitroanilide (20 µL) serving as an artificial, synthetic substrate was added to the plate, and incubation was performed at 37°C for 24 hours. Thereafter, the absorbance was measured at 405 nm. p-Nitroaniline was used as a standard, and the elastase activity of the skin homogenate was obtained. Fig. 3 shows the results.

As shown in Fig. 3, the control group exhibited considerably increased elastase activity after exposure to UV light, as compared with that of the blank group. However, the bacterium cell group exhibited suppressed elastase activity as compared with that of the control group.

### Example 4 (Preparation of bacterium cell sample)

A medium was prepared from mineral solution (1 w/v%), yeast extract (1 w/v%), lactose (3 w/v%), and milk protein (5 w/v%). The medium (1.5 L) was added to a 2L flask and sterilized by heating at 121°C for 15 minutes. To the sterilized medium, cells of *Bifidobacterium breve* YIT 12272 (FERM BP-11320) were inoculated at 1 v/v%, and anaerobically cultured at 36°C for about 20 hours, while the pH of the culture was maintained at 5.5 by use of sodium hydroxide. The thus-obtained culture liquid was centrifuged at 15,000×G, to thereby recover cells of a bacterium belonging to the genus *bifidobacterium.* The above mineral solution had the following composition: potassium phosphate(I) (10 w/v%), potassium phosphate(II) (20 w/v%), sodium acetate (30 w/v%), and ammonium sulfate (30 w/v%).

Separately, a dispersion (100 mL) of milk protein (8 w/v%) and sugar (4 w/v%) was prepared and sterilized by heating at 121°C for 15 minutes. To the dispersion, the above collected cells of the bacterium belonging to the genus *bifidobacterium* were dispersed at 15% (based on wet weight) and then lyophilized, to thereby yield freeze-dried cells of *Bifidobacterium breve* YIT 12272 (FERM BP-11320). The freeze-dried cells were suspended in physiological saline (10 mL), to thereby prepare a bacterium cell sample having a cell concentration of 4 × 10⁹ CFU/mL.

### Test Example 4 (Elastase activity suppression test)

Hair-less mice (Hos: HR-1 (6-week-old)) were acclimated for one week and divided into three groups (n=6): group 1 to group 3.

Physiological saline was perorally administered to groups 1 and 2. To group 3, a bacterium cell sample prepared in Example 4 was perorally administered. In all cases, the solution was administered to mice at a dose of 0.1 mL/day for nine days.

Group 1 was not exposed to UV light. Groups 2 and 3 were irradiated with UV light (including 280 to 400 nm) at a daily dose of 50 mJ/cm² by means of a UV-radiation apparatus (Toshiba SE-FL-20) for four days from day 6 after start of administration, while the administration was continued. Then, from each mouse, the dorsal skin was removed.

Among the mouse groups, a group to which physiological saline was administered but which was not irradiated with UV light was a blank group; a group to which physiological saline was administered and which was irradiated with UV light was a control group; and a group to which a bacterium cell sample was administered and which was irradiated with UV light was a bacterium cell group (Table 4). In the following Table 4, "UV+" denotes performing UV irradiation, and "UV-" denotes performing no UV irradiation.

**[Table 4]**

| | |
|---|---|
| Group 1 | Blank group (UV-, physiological saline) |
| Group 2 | Control group (UV+, physiological saline) |
| Group 3 | Bacterium cell group (UV+, bacterium cells) |

To each of the thus-collected dorsal skin samples, PBS (phosphate buffered physiological saline) was added, and the mixture was homogenized by means of Polytron. The homogenate was centrifuged at 14,000×G, and the supernatant was dispensed to a 96-well plate (each 100 µL). Instead of elastin, 6.25 mM succinyl-L-alanyl-L-alanyl-L-alanine-p-nitroanilide (20 µL) serving as an artificial, synthetic substrate was added to the plate, and incubation was performed at 37°C for 24 hours. Thereafter, the absorbance was measured at 405 nm. p-Nitroaniline was used as a standard, and the elastase activity of the skin homogenate was obtained. Fig. 4 shows the results.

As shown in Fig. 4, the control group exhibited considerably increased elastase activity after exposure to UV light, as compared with that of the blank group. However, the bacterium cell group exhibited suppressed elastase activity as compared with that of the control group.

## Claims

1. A *Bifidobacterium breve* bacterium for use in a method of, by oral application, preventing or treating skin cancer by promoting DNA damage repair or by promoting reduction of cyclobutane pyrimidine dimer level.

2. *The Bifidobacterium breve* bacterium for the use according to claim 1, wherein the *Bifidobacterium breve* bacterium is *Bifidobacterium breve* YIT 4065 or *Bifidobacterium breve* YIT 12272.

3. The *Bifidobacterium breve* bacterium for the use according to claim 1, wherein the DNA damage repair is repair of DNA damage in the skin caused by exposure to UV light.

4. The *Bifidobacterium breve* bacterium for the use according to claim 3, wherein said *Bifidobacterium breve* bacterium is perorally administered at least before exposure to UV light.

5. A non-therapeutic method for skin anti-aging or skin revitalization by suppressing elastase activity, comprising perorally administering to a subject in need thereof a *Bifidobacterium breve* bacterium that is *Bifidobacterium breve* YIT 12272.

6. The method according to claim 5, which suppresses the activity of elastase in the skin enhanced by exposure to UV light.

7. The *Bifidobacterium breve* bacterium for use or the method according to any one of claims 1 to 6, wherein said *Bifidobacterium breve* bacterium is administered in an amount of 1 × 10³ CFU or more as a daily dose.

8. The *Bifidobacterium breve* bacterium for use or the method according to any one of claims 1 to 7, wherein said *Bifidobacterium breve* bacterium is administered for five days or longer before exposure of said subject to UV light.

## Patentansprüche

1. *Bifidobacterium breve*-Bakterium zur Verwendung in einem Verfahren zur Verhinderung oder zur Behandlung von Hautkrebs durch orale Verabreichung, indem die Reparatur von DNA-Schäden gefördert wird oder indem die Reduktion des Spiegels an Cyclobutanpyrimidin-Dimer gefördert wird.

2. *Bifidobacterium breve*-Bakterium zur Verwendung nach Anspruch 1, wobei das *Bifidobacterium breve*-Bakterium *Bifidobacterium breve* YIT 4065 oder *Bifidobacterium breve* YIT 12272 ist.

3. *Bifidobacterium breve*-Bakterium zur Verwendung nach Anspruch 1, wobei die Reparatur der DNA-Schäden die Reparatur von DNA-Schäden in der Haut ist, die durch Bestrahlung mit UV-Licht hervorgerufen werden.

4. *Bifidobacferium breve*-Bakterium zur Verwendung nach Anspruch 3, wobei das *Bifidobacterium breve*-Bakterium peroral wenigstens vor der Bestrahlung mit UV-Licht verabreicht wird.

5. Nicht-therapeutisches Verfahren gegen Hautalterung oder für die Revitalisierung der Haut durch Unterdrücken der Elastase-Aktivität, umfassend das perorale Verabreichen eines *Bifidobacterium breve*-Bakteriums, das *Bifidobacterium breve* YIT 12272 ist, an eine Person, die dessen bedarf.

6. Verfahren nach Anspruch 5, in welchem die Elastase-Aktivität in der Haut unterdrückt wird, die durch Bestrahlung mit UV-Licht verstärkt wird.

7. *Bifidobacterium breve*-Bakterium zur Verwendung oder das Verfahren nach einem der Ansprüche 1 bis 6, wobei das *Bifidobacterium breve*-Bakterium in einer Menge von 1 x 10³ KbE (CFU) oder mehr als tägliche Dosis verabreicht wird.

8. *Bifidobacterium breve*-Bakterium zur Verwendung oder das Verfahren nach einem der Ansprüche 1 bis 7, wobei das *Bifidobacterium breve*-Bakterium für fünf Tage oder länger vor der Bestrahlung der Person mit UV-Licht verabreicht wird.

## Revendications

1. Bactérie *Bifidobacterium breve* pour une utilisation dans une méthode, par application par voie orale, de prévention ou de traitement d'un cancer de la peau, par promotion de la réparation d'un endommagement de l'ADN ou par promotion d'une réduction du niveau de dimère de cyclobutane-pyrimidine.

2. Bactérie *Bifidobacterium breve* pour une utilisation selon la revendication 1, laquelle bactérie *Bifidobacterium breve* est *Bifidobacterium breve* YIT 4065 ou *Bifidobacterium breve* YIT 12272.

3. Bactérie *Bifidobacterium breve* pour une utilisation selon la revendication 1, dans laquelle la réparation d'un endommagement de l'ADN est une réparation d'un endommagement de l'ADN dans la peau dû à une exposition à une lumière UV.

4. Bactérie *Bifidobacterium breve* pour une utilisation selon la revendication 3, laquelle bactérie *Bifidobacterium breve* est administrée par voie orale au moins avant exposition à une lumière UV.

5. Méthode non thérapeutique pour lutter contre le vieillissement de la peau ou pour revitaliser la peau par suppression de l'activité d'élastase, comprenant l'administration par voie orale, à un sujet en ayant besoin d'une bactérie *Bifidobacterium breve* qui est *Bifidobacterium breve* YIT 12272.

6. Méthode selon la revendication 5, qui supprime l'activité d'élastase dans la peau amplifiée par une exposition à une lumière UV.

7. Bactérie *Bifidobacterium breve* pour une utilisation ou méthode selon l'une quelconque des revendications 1 à 6, dans laquelle ladite bactérie *Bifidobacterium breve* est administrée en une quantité correspondant à une dose journalière de 1 x 10³ CFU ou plus.

8. Bactérie *Bifidobacterium breve* pour une utilisation ou méthode selon l'une quelconque des revendications 1 à 7, dans laquelle ladite bactérie *Bifidobacterium breve* est administrée pendant cinq jours ou plus avant une exposition dudit sujet à une lumière UV.
